Europäisches Patentamt

European Patent Office

Office européen des brevets

(19)

(11) Publication number: **0 337 739**
**A2**

# EUROPEAN PATENT APPLICATION

(12)

(21) Application number: **89303581.6**

(22) Date of filing: **11.04.89**

(51) Int. Cl.⁴: **A 61 K 31/445**
**A 61 K 31/55, A 61 K 31/00**

(30) Priority: **12.04.88 GB 8808599**

(43) Date of publication of application:
**18.10.89 Bulletin 89/42**

(84) Designated Contracting States:
**AT BE CH DE ES FR GB GR IT LI LU NL SE**

(71) Applicant: **GLAXO GROUP LIMITED**
**Clarges House 6-12 Clarges Street**
**London W1Y 8DH (GB)**

(72) Inventor: **Naylor,Ian Leslie**
**45 Heaton Grove**
**Bradford (GB)**

(74) Representative: **Watkins, Arnold Jack et al**
**European Patent Attorney Frank B. Dehn & Co. Imperial**
**House 15-19 Kingsway**
**London WC2B 6UZ (GB)**

(54) Use of TP-receptor antagonists to modify the action of myofibroblasts.

(57) The use is described of TP-receptor antagonists in the manufacture of medicaments for the treatment or prophylaxis of conditions caused or exacerbated by myofibroblast cell contraction.

EP 0 337 739 A2

## Description

## USE OF TP-RECEPTOR ANTAGONISTS TO MODIFY THE ACTION OF MYOFIBROBLASTS

This invention relates to a new medical use for certain chemical compounds and pharmaceutical compositions containing them. In particular, it relates to the use of TP-receptor antagonists to modify the action of myofibroblasts.

A myofibroblast is a cell type which has the structural and functional properties of both a fibroblast and a smooth muscle cell. Myofibroblasts are found in a variety of human and animal tissues and proliferate in various "disease" states ranging from wound contracture to many types of cancer.

The ability of myofibroblasts to contract like smooth muscle cells is a valuable property utilised in the natural healing processes where tissue damage has occurred. In particular, the myofibroblasts congregate at the site of injury. They then begin to contract and this helps to contain and reduce the size of the injury. Contraction of the myofibroblast cells also acts as a stimulus for the formation of other myofibroblasts which are then produced at the site of injury and exert an even greater contractile effect.

Unfortunately, situations may arise, particularly when tissue damage has occurred, when myofibroblast accumulation and proliferation at a tissue site leads to excessive contraction. This, in turn, often leads to undesirable effects for which, to date, no treatment has been available. Conditions caused or exacerbated by myofibroblast cell contraction include keloid formation, hypertrophic scars and wound contracture after injury (especially burn injury), post-operative strictures around orifices, capsular contractions around implants, scar tissue formation during nerve regeneration, Dupuytren's Contracture, contraction in the palate after injury, intraocular damage after penetration eye injury, contraction of skin grafts and portal hypertension associated with liver damage (e.g. liver cirrhosis).

We have now found suprisingly that compounds which act as TP-receptor antagonists are of benefit in modifying the action of myofibroblasts. More particularly, we have found that TP-receptor antagonists inhibit or reduce the contractibility of myofibroblasts and may therefore be of use in human and veterinary medicine for the treatment or prophylaxis of conditions caused or exacerbated by myofibroblast cell contraction.

Thus, according to one aspect of the invention, we provide a TP-receptor antagonist for use in the manufacture of a medicament for the treatment or prophylaxis of conditions caused or exacerbated by myofibroblast cell contraction.

In a further aspect of the present invention, we provide a method of treatment of a human or animal subject susceptible to conditions caused or exacerbated by myofibroblast cell contraction which method comprises administering to the subject a TP-receptor antagonist in an amount sufficient to inhibit or reduce the contractibility of myofibroblast cells.

In another aspect of the present invention, we provide the use of a TP-receptor antagonist for the treatment or prophylaxis of conditions causes or exacerbated by myofibroblast cell contraction.

Conditions caused or exacerbated by myofibroblast cell contraction which may be improved by administration of a TP-receptor antagonist include keloid formation, hypertrophic scars and wound contracture after injury (especially burn injury), post-operative strictures around orifices, capsular contractions around implants, scar tissue formation during nerve regeneration, Dupuytren's Contracture, contracture in the palate after injury, intraocular damage after penetration eye injury, contraction of skin grafts and portal hypertension associated with liver damage (e.g. liver cirrhosis). Administration of a TP-receptor antagonist might also enable the more rapid expansion of a tissue expander device by minimising the resistance to expansion caused by the myofibroblasts in the capsule formed around the device after implantation.

In a particular embodiment of the present invention, we provide a TP-receptor antagonist for use in the manufacture of a medicament for the treatment or prophylaxis of conditions caused or exacerbated by myofibroblast cell contraction at a site of tissue damage.

In a further particular embodiment of the present invention, we provide a method of treatment of a human or animal subject with tissue damage which method comprises administering to the subject a TP-receptor antagonist in an amount sufficient to inhibit or reduce the contractibility of myofibroblast cells at the site of tissue damage.

It should be understood that it may be appropriate to treat the skin graft (especially when a split-skin graft or a thin-split graft is used) or suitable constituents of a tissue expander with the TP-receptor antagonist prior to the skin graft or tissue expander implant operation.

Thus, in a further embodiment of the present invention, we provide a skin graft including a TP-receptor antagonist in an amount sufficient to inhibit or reduce the contractibility of myofibroblast cells at the site of the skin graft operation.

In a yet further embodiment of the present invention, we provide a tissue expander device including a TP-receptor antagonist in an amount sufficient to inhibit or reduce the contractibility of myofibroblast cells at the site of implant. In a preferred embodiment, the TP-receptor antagonist is incorporated into the plastic constituents of a tissue expander device.

It is preferable to employ the TP-receptor antagonist according to the present invention in the form of a pharmaceutical formulation. The present invention therefore also provides a pharmaceutical composition for use in human or veterinary medicine for the treatment or prophylaxis of conditions caused or exacerbated by myofibroblast cell contraction comprising a TP-receptor antagonist together, where desirable, with one or more pharmaceutical carriers or excipients.

The term 'TP-receptor antagonist' means herein any compound which blocks the receptor through which thromboxane A2 induces its effects. This receptor has been called a TP-receptor in the general classification of prostanoid receptors [Kennedy et al, Prostaglandins, 24, 667 (1982)]. Activity for TP-receptor blocking drugs has been found in a wide variety of experimental models including myocardial infarction, coronary thrombosis and other models of platelet deposition, ischaemia and reperfusion arrhythmias, cerebral vasospasm, proteinurea, endotoxin shock and antigen-induced bronchoconstriction. However, the present invention for the first time links TP-receptor blocking activity with modification of the action of myofibroblasts.

The efficacy of TP-receptor antagonists in controlling the action of myofibroblasts may be demonstrated in in vitro studies, for example by determining their ability to block the contractile effect of U-46619, a potent and stable thromboxane agonist (see Prostaglandins and Related Substances - a Practical Approach, Ed. C. Benedetto, R. G. McDonald Gibson, S. Nigam and T. F. Slater, 1987, IRL Press, Oxford and Washington DC., especially Chapter 16 entitled "Methods in prostanoid receptor classification" by R. A. Coleman), on strips of human tissue containing high concentrations of myofibroblasts. Examples of suitable tissue include granulation tissue, keloids, tissue expander capsules, Dupuytren's nodules and cords and hypertrophic scars, and experiments may be carried out using the standard organ bath technique or using the technique of superfusion (see R. A. Coleman et al., Br. J. Pharmac. (1981), 73, 773-778; s.a. J. H. Gaddum, Brit. J. Pharmacol. (1953), 8, 321-326).

Examples of TP-receptor antagonist compounds which may find use according to the present invention are those disclosed in EP-A-32432, EP-A-43292, EP-A-44711, EP-A-62918, EP-A-69810, EP-A-74861, EP-A-82646, EP-A-94080, EP-A-94239, EP-A-94792, EP-A-107995, EP-A-126088, EP-A-127930, EP-A-137426, EP-A-140307, EP-A-140684, EP-A-142322, EP-A-142324, EP-A-145260, EP-A-150015, EP-A-150709, EP-A-150710, EP-A-156245, EP-A-159528, EP-A-161904, EP-A-162762, EP-A-163562, EP-A-171146, EP-A-183238, EP-A-201349, EP-A-201350, EP-A-201351, EP-A-201352, EP-A-201354, EP-A-201811, EP-A-202084, EP-A-202086, EP-A-205834, EP-A-205857, EP-A-207684, EP-A-210664, EP-A-220848, EP-A-221344, EP-A-223518, EP-A-226340, EP-A-228887, EP-A-232912, EP-A-234708, EP-A-239907, EP-A-242518, EP-A-247089, EP-A-251011, EP-A-253257, EP-A-253321, EP-A-262395, EP-A-266980, EP-A-270929, EP-A-284892, EP-A-287270, EP-A-288279, EP-A-289911, EP-A-290285, EP-A-296802, EP-A-297768, GB-A-2028805, GB-A-2070588, GB-A-2075503, GB-A-2079281, GB-A-2084150, GB-A-2086384, GB-A-2097397, GB-A-2103608, GB-A-2119375, GB-A-2122997, GB-A-2127406, GB-A-2132199, GB-A-2133014, GB-A-2133791, GB-A-2136428, GB-A-2145715, GB-A-2146640, GB-A-2146023, GB-A-2148293, GB-A-2149786, GB-A-2153352, GB-A-2158064, GB-A-2159816, GB-A-2162512, GB-A-2165235, GB-A-2165535, GB-A-2167402, GB-A-2167403, GB-A-2167404, GB-A-2168343, GB-A-2169899, GB-A-2171098, GB-A-2171405, GB-A-2173796, GB-A-2173797, GB-A-2174707, GB-A-2177091, GB-A-2177398, GB-A-2178430, GB-A-2183234, GB-A-2190083, J58013548A, J58013551A, J60004154A, J63101322A, J63183574A, J63225375A, J63284158A, US Patent Nos. 4239778, 4258058, 4443477, 4536510, 4542160, 4555520, 4555523, 4582854, 4588741, 4591603, 4608386, 4611006, 4626548, 4632931, 4638012, 4647573, 4652578, 4654357, 4654364, 4663337, 4673685, 4675405, 4734424, 4734425, 4734426, 4735962, 4738978, 4743697, 4749715, 4752613, 4775680, 4783473, German Offenlegungschrift Nos. 3518271 and 3539218, WO-A-8704428 and WO-A-8803137.

Particular mention may be made of the compounds which act as TP-receptor antagonists disclosed in EP-A-32432, EP-A-74861, EP-A-127930, EP-A-296802, GB-A-2028805, GB-A-2075503, GB-A-2079281, GB-A-2097397, GB-A-2127406, GB-A-2146023, GB-A-2159816, GB-A-2165235, GB-A-2165535, GB-A-2167402, GB-A-2167403, GB-A-2167404 and GB-A-2183234.

The contents of the above mentioned patents and published patent applications are incorporated by reference herein.

Specific compounds for use according to the present invention include :
13-azaprostanoic acid;
[2α,4α,5α(Z)]-6-[2-(2-chlorophenyl)-4-(2-hydroxyphenyl)-1,3-dioxan-5-yl]-4-hexenoic acid;
[1S-[1α,2β,(Z),3α(S*),5α]]-7-[3-[(cyclopentylhydroxyacetyl)amino]-6,6-dimethylbicyclo[3.1.1]hept-2-yl]-5-heptenoic acid;
[1S-[1α,2α,(Z),3α,4α]]-7-[3-[[[[(1-oxoheptyl)amino]acetyl]amino]methyl]-7-oxabicyclo[2.2.1]hept-2-yl]-5-heptenoic acid;
[1α,2β(Z),3α,4α]-(±)-7-[3-[1-[[(phenylamino)thioxomethyl] hydrazono]ethyl]bicyclo[2.2.1]hept-2-yl]-5-heptenoic acid;
[p-(2-benzenesulphonamidoethyl)phenoxy]acetic acid;
4-[2-(4-chlorobenzenesulphonylamino)ethyl]benzeneacetic acid;
[1α(Z),2β,5β]-(±)-6-[[2-(hexahydro-1H-azepin-1-yl)-5-[[2'-(hydroxymethyl)[1,1'-biphenyl]-4-yl]methoxy]cyclopentyl]oxy]-4-hexenoic acid and the 1R and 1S isomers thereof; and
[1R-[1α(Z),2β,3β,5α]]-(+)-7-[5-[[(1,1'-biphenyl)-4-yl]methoxy]-3-hydroxy-2-(1-piperidinyl)cyclopentyl]-4-heptenoic acid (hereinafter referred to as 'Compound A'), and their physiologically acceptable salts and solvates, and cyclodextrin complexes of Compound A.

A particularly preferred compound for use according to the present invention is Compound A or a physiologically acceptable salt, solvate or cyclodextrin complex thereof. A particularly preferred form of Compound A is its hydrochloride salt. Another particularly preferred form of Compound A is its β-cyclodextrin

3

complex in which the molar ratio of Compound A to β-cyclodextrin is about 1:1.

Other particularly preferred compounds for use according to the present invention are [1α,(Z),2β,5β]--(±)-6-[[2-(hexahydro-1H-azepin-1-yl)-5-[[2′-(hydroxymethyl)[1,1′-biphenyl]-4-yl]methoxy]cyclopentyl] oxy-4-hexenoic acid (hereinafter referred to an 'Compound B') and the 1R and 1S isomers thereof and their physiologically acceptable salts, solvates and cyclodextrin complexes.

13-Azaprostanoic acid is a known compound described by Venton et al. in J. Med. Chem. 22, 82 (1979). [p-(2-Benzenesulphonamidoethyl)-phenoxy]acetic acid is described by Patscheke and Stagmeier in Thromb. Res. 33, 277 (1984) and 4-[2-(4-chlorobenzenesulphonylamino)ethyl]-benzeneacetic acid is described by Patscheke et al. in Thromb. Haemostasis 58, 182 (1987). [2α,4α,5α(Z)]-6-[2-(2-chlorophenyl)-4-(2-hydroxyphenyl)-1,3-dioxan-5-yl-4-hexenoic acid is described by A. G. Brewster et al. in Prostaglandins 36(2), 173-178. [1S-[1α,2β(Z),3α(S*),5α]]-7-[3-[(cyclopentylhyroxyacetyl)amino]-6,6-dimethylbicyclo[3.1.1]hept-2-yl]-5-heptenoic acid is described by M. Fujioka et al. in Arch. Pharmacol. 334(4), 468-474. [1S-[1α,2α(Z),3α,4α]]--7-[3-[[[[(1-oxoheptyl)amino]acetyl]amino]methyl]-7-oxabicyclo[2.2.1]hept-2-yl]-5-heptenoic acid is described by G. J. Grover et al. in J. Cardiovasc. Pharmacol. 12(6), 701-709. [1α,2β(Z),3α,4α]-(±)-7-[3-[1-[[(phenylamino)thioxomethyl]hydrazono]ethyl]bicyclo[2.2.1]hept-2-yl]-5-heptenoic acid is described by R. A. Armstrong et al. in Br. J. Pharmacol. 84(3), 595-607.

The TP-receptor antagonists described herein may be used according to the present invention with other suitable therapeutic agents such as, for example, thromboxane synthase inhibitors, and it is to be understood that this invention covers such combinations when used to modify the action of myofibroblasts.

Compound A and physiologically acceptable salts and solvates thereof are described in GB-A-2097397 and GB-A-2127406. Cyclodextrin complexes of Compound A are described in our co-pending UK Patent Applications Nos. 8729823 and 8804422. The cyclodextrin complexes may be prepared by dissolving Compound A or the hydrochloride salt thereof in water or an organic solvent which is miscible with water (e.g. an alcohol such as methanol) and adding to the solution a solution ofα-, β- or γ-cyclodextrin (or a hydrate thereof) or a mixture of two or three of them together in water and/or an organic solvent which is miscible with water. The reaction may take place at any temperature in the range from 0° to 80°C. However, the mixture is preferably kept at room temperature and the desired complex obtained by concentrating the mixture under reduced pressure or by allowing the mixture to cool. The mixing ratio of organic solvent with water may be suitably varied according to the solubilities of the starting materials and products. Preferably 1 to 4 moles of cyclodextrin are used for each mole of Compound A or its hydrochloride salt.

Compound B and physiologically acceptable salts, solvates and cyclodextrin complexes thereof are described in EP-A-296802. The 1R and 1S isomers of Compound B may be prepared according to the general methods described in EP-A-296802 from starting materials having the desired stereochemical configuration. Examples of the preparation of the 1R and 1S isomers of Compound B are described hereinafter.

Pharmaceutical compositions for use according to the present invention may be formulated in conventional manner, optionally with one or more physiologically acceptable carriers and/or excipients. For example, the compounds described in the aforementioned patent specifications and literature publications may be formulated in the manner described therein. The compositions may be administered by any suitable method known in the medicinal arts. Suitable modes of administration include, but are not limited to, oral, parenteral (e.g. intramuscular, subcutaneous, intraperitoneal or intravenous) or topical administration or administration by inhalation.

Pharmaceutical formulations containing a cyclodextrin complex of Compound A may be prepared in conventional manner by mixing the complex with one or more pharmaceutical carriers or excipients, for example, according to the general methods described in GB-A-2097397 and GB-A-2127406.

When Compound A or a physiologically acceptable salt, solvate or cyclodextrin complex is to be administered as an aqueous formulation for, in particular, parenteral (e.g. intravenous) use, the composition may be prepared according to the general methods described in GB-A-2097397 and GB-A-2127406. Alternatively, the aqueous compositions may be prepared by mixing Compound A or, more preferably, the hydrochloride salt thereof with α-, β- or γ-cyclodextrin (or a hydrate thereof) or a mixture of two or three of them together, where desirable, with one or more pharmaceutical carriers or excipients in a suitable medium such as water according to conventional methods and, for example, as described in the Examples hereinafter. Preferably, Compound A or its hydrochloride salt are dissolved in water and the remaining constituents are added thereto.

The molar ratio of Compound A or its hydrochloride salt with cyclodextrin in the liquid composition is conveniently within the range 1:1 to 1:3.

Preferably, the aqueous formulation comprises the hydrochloride salt of Compound A and β-cyclodextrin (or a hydrate thereof) at about physiological pH wherein the formulation contains at least about 1.2 moles of β-cyclodextrin (e.g. 1.2 to 2 moles) for every one of the hydrochloride salt of Compound A. Preferably the molar ratio of the hydrochloride salt of Compound A to β-cyclodextrin is about 1:1.4.

For topical administration the TP-receptor antagonists may be formulated as ointments, creams, lotions, powders, pessaries, sprays, aerosols or drops (e.g. eye or nose drops). Ointments and creams may, for example, be formulated with an aqueous or oily base with the addition of suitable thickening and/or gelling agents. Lotions may be formulated with an aqueous or oily base and will, in general, also contain one or more emulsifying, dispersing, suspending, thickening or colouring agents. Powders may be formed with the aid of any suitable powder base. Drops may be formulated with an aqueous or non-aqueous base also comprising one or more dispersing, solubilising or suspending agents. Aerosol sprays are conveniently delivered from pressurised packs, with the use of a suitable propellant.

The precise dose of the TP-receptor antagonist to be administered and the length of the course of treatment will, of course, depend on a number of factors including, for example, the age and weight of the patient, the specific condition requiring treatment and its severity and the route of administration. Generally however the dosages and dosage rates stated in the aforementioned patent specifications and literature publications as being effective in treating other conditions may be suitable for the treatment or prophylaxis of conditions caused or exacerbated by myofibroblast cell contraction. Thus, for example, in the case of Compound A or a physiologically acceptable salt, solvate or cyclodextrin complex thereof, an effective dose is likely to be in the range from 0.05 to 20 mg/kg body weight of patient per day, preferably in the range from 0.05 to 5 mg/kg per day.

The ability of Compound A to block the contractile effect of U-46619 on strips of Dupuytren's nodules has recently been reported by D. J. Coleman et al. in Br. J. Pharmac. (1989), 96, 65P wherein Compound A is referred to by its code number GR 32191.

The intermediates and Examples 1 and 2 relate to the preparation of the 1R and 1S isomers of Compound B. The remaining examples illustrate pharmaceutical formulations for use according to the present invention containing Compound A as the active ingredient. Other TP-receptor antagonists may be formulated in the manner described in the aforementioned patent specifications and literature publications relating thereto.

Intermediate 1

(±)-1-Bromo-4-[[(2-cyclopenten-1-yl)oxy]methyl]benzene

A mixture of the 2-cyclopenten-1-ol (J. L. Eisch et. al, J. Org. Chem. 44, 587 (1979) (6g), tetrabutylammonium hydrogensulphate (1g), 4-bromobenzyl bromide (23g) in dichloromethane (100mℓ) and 70% sodium hydroxide solution (40mℓ) was stirred at 20° for 3 days. A further quantity (7.5g) of the bromide was added and stirring continued for a further 24h. The mixture was diluted with water and dichloromethane and the layers were separated. The aqueous layer was extracted with dichloromethane and the organic extracts were washed with water, dried and evaporated to give an oil which was purified by flash column chromatography on silica eluting initially with petroleum ether (b.p. 40°-60°) then with 19:1 petroleum ether (b.p. 40°-60°) - ether to give the title compound as a pale-yellow oil (14.1g).

| Analysis Found: | C,56.7; | H,5.0. |
| --- | --- | --- |
| $C_{12}H_{13}BrO$ requires | C,56.9; | H,5.2%. |

Intermediate 2

(1α,2α,5α)-(±)-2-[(4-Bromophenyl)methoxy]-6-oxabicyclo[3.1.0]hexane

m-Chloroperbenzoic acid (85%, 13.4g) was added over 1.5h to a cold (0°) stirred solution of Intermediate 1 in dichloromethane (200mℓ). The mixture was stirred at ambient temperature for 16h, filtered and the filtrate washed with a solution of potassium carbonate (150g) and sodium sulphite (50g) in water (500mℓ). The aqueous layer was extracted with dichloromethane and the combined organic extracts were dried and evaporated. The residue was purified by flash column chromatography on silica using petroleum ether (b.p. 40°-60°) - ether (3:1) as eluent to give the title compound as an oil (5.64g).

| Analysis Found: | C,53.32; | H,4.83. |
| --- | --- | --- |
| $C_{12}H_{13}BrO_2$ requires | C,53.55; | H,4.87%. |

Intermediate 3

(1α,2β,5β)-(±)-2-[(4-Bromophenyl)methoxy]-5-(hexahydro-1H-azepin-1-yl)-cyclopentan-1-ol

A solution of Intermediate 2 (5.43g) and hexamethyleneimine (25mℓ) in butan-1-ol (75mℓ) were heated at reflux for 22.5h. Evaporation of the solvent and excess hexamethyleneimine in vacuo gave a residue which was purified by flash column chromatography on triethylamine deactivated silica with ethyl acetate:methanol (9:1) as the eluent to give the title compound as a pale brown oil (6.6g).

Analysis Found: C,59.0;     H,7.3;     N,4.15.
$C_{18}H_{26}BrNO_2$   C,58.7;     H,7.1;     N,3.8%.
requires

## Intermediate 4

[1R-[1α,2β,5β]]-2-[(4-Bromophenyl)methoxy]-5-(hexahydro-1H-azepin-1-yl)cyclopentan-1-ol (D)-dibenzoyl tartaric acid salt and
[1S-[1α,2β,5β]]-2-[(4-bromophenyl)methoxy]-5-(hexahydro-1H-azepin-1-yl)cyclopentan-1-ol (L)-dibenzoyl tartaric acid salt

A mixture of Intermediate 3 (15g) and (L)-dibenzoyl tartaric acid (15.33g) was heated in ethyl acetate (200ml) with addition of methanol until dissolved. The mixture was allowed to cool whereupon a white solid crystallised which was collected by filtration. This solid was recrystallised from 10% methanol in ethyl acetate (with addition of methanol until totally dissolved). On standing in a fridge for 36h large clumps of off-white crystals formed, which were collected and dried under vacuum to give the title (1S) salt (9.6g), m.p. 158° dec.

The mother liquors from the above reaction were basified (2N sodium hydroxide solution) and extracted into ether, dried (MgSO₄) and concentrated. The concentrate and (D)-dibenzoyltartaric acid (11.45g) were dissolved in an ethyl acetate:methanol (10:1) mixture at reflux, with addition of methanol to complete dissolution. On cooling in a fridge (56h) off-white crystals formed. These were collected by filtration and dried under vacuum to give the title (1R) salt (10.02g) m.p. 155° dec.

## Intermediate 5

[1R-[1α,2β,5β]]-2-[(4-Bromophenyl)methoxy]-5-(hexahydro-1H-azepin-1-yl)cyclopentan-1-ol

Sodium hydroxide (2N; 75ml) was added to the (1R) salt of Intermediate 4 (4.04g) and the mixture stirred until the salt had dissolved. The phases were separated and the aqueous extracted with dichloromethane (50ml), combined organic phases dried (MgSO₄) and evaporated to give the title compound as a colourless oil (2.08g). T.l.c. SiO₂ [ether:hexane (3:1);Et₃N doped] Rf 0.04. $[\alpha]_D^{20} = +1.72°$ (ethanol).

## Intermediate 6

[1S-[1α,2β,5β]]-2-[(4-Bromophenyl)methoxy]-5-hexahydro-1H-azepin-1-yl) cyclopentan-1-ol

Sodium hydroxide (2N;75ml) was added to the (1S) salt of Intermediate 4 (4.04g) in dichloromethane (50ml) and the mixture stirred until the salt had dissolved. The phases were separated and the aqueous extracted with dichloromethane (50ml). Combined organic phases were dried (MgSO₄) and evaporated to give the title compound (2.11g) as a colourless oil. T.l.c. SiO₂ [ether:hexane (3:1);Et₃N doped] Rf 0.04. $[\alpha]_D^{20} = -1.64°$ (ethanol).

## Intermediate 7

[1R-[1α,(Z),2β,5β]]-1,1'Dimethylethyl
6-[[2-[4-bromophenyl)methoxy]-5-(hexahydro-1H-azepin-1-yl)cyclopentyl]oxy]-4-hexenoate

To a solution of Intermediate 5 (2.02g) in dry dimethylformamide under nitrogen was added sodium hydride (80% in oil, 263mg). The mixture was warmed until vigorous hydrogen evolution occurred. When effervescence had ceased the suspension was cooled to 0° and (Z)-1,1-dimethylethyl 6-chloro-4-hexenoate (1.8ml) was added. The mixture was stirred for 2h, diluted with water (100ml) and extracted with ether (3 x 50ml). Combined extracts were washed with water, dried (MgSO₄) and evaporated to give a solid. Purification by chromatography [Fluka HF₂₅₄ SiO₂ pretreated with triethylamine] eluting with ether:hexane (3:1) → ethyl acetate:ethanol (1:1) gave the title compound (1.28g) as a light yellow oil. T.l.c. SiO₂ [ether:hexane (3:1): Et₃N doped] Rf 0.35. $[\alpha]_D^{20} = -0.37°$ (ethanol).

## Intermediate 8

[1S-[1α(Z),2β,5β]]-1,1'-Dimethylethyl
6-[[2-[(4-bromophenyl)methoxy]-5-(hexahydro-1H-azepin-1-yl)cyclopentyl]oxy]-4-hexenoate

To a solution of Intermediate 6 (2.05g) in dry dimethylformamide (20ml) was added sodium hydride (80% in oil, 270mg). The mixture under nitrogen was heated to ca. 60° until hydrogen evolution ceased. (Z)-1,1-dimethylethyl 6-chloro-4-hexenoate (1.82ml) was added dropwise to the solution at 0° and stirred for 2h. Water (50ml) and brine (50ml) were added and extracted with ethyl acetate (3 x 50ml). Combined extracts were washed with water, dried (MgSO₄) and evaporated to a solid. Purification by chromatography [Fluka HF₂₅₄ SiO₂ pretreated with triethylamine] eluting with ether:hexane (3:1) → ether:ethanol (4:1) gave the title compound (780mg) as a colourless oil. T.l.c. SiO₂ [ether:hexane (3:1); Et₃N doped] Rf 0.35. $[\alpha]_D^{20} = +0.52°$ (ethanol).

Intermediate 9

[1R-[1α(Z),2β,5β]]-6-[[2-[(4-Bromophenyl)methoxy]-5-(hexahydro-1H-azepin-1-yl)cyclopentyl]oxy]-4-hexenoic acid
To a solution of Intermediate 7 (1.24g) in ethanol (15ml) was added sodium hydroxide solution (2N;5ml). The solution was allowed to stand at room temperature for 24h, heated at reflux for 2h, neutralized with 2N hydrochloric acid and diluted with pH 6.5 buffer (50ml). The aqueous mixture was extracted with dichloromethane (3 x 50ml). Combined extracts were dried (MgSO$_4$) and evaporated to give the title compound (1.16g) as a colourless gum. T.l.c. SiO$_2$ [ethyl acetate:ethanol (2:1), 1% NH$_3$] Rf 0.1. [α]$_D^{20}$ = + 38.98° (chloroform).

Intermediate 10

[1S-[1α(Z),2β,5β]]-6-[[2-[(4-Bromophenyl)methoxy]-5-(hexahydro-1H-azepin-1-yl)cyclopentyl]oxy]-4-hexenoic acid
To a solution of Intermediate 8 (743mg) in ethanol (10ml) was added sodium hydroxide solution (2N;3ml) and the mixture allowed to stand at room temperature for 24h, then heated at reflux for 2h. The mixture was neutralised (2N HCl), diluted with pH 6.5 buffer (50ml) and extracted with dichloromethane (3 x 50ml). Combined extracts were dried (MgSO$_4$) to give the title compound (392mg) as a colourless gum. T.l.c. SiO$_2$ [ethyl acetate:ethanol (2:1), 1% NH$_3$] Rf 0.1. [α]$_D^{20}$ = -37.18° (chloroform).

Intermediate 11

(Z)-1,1-Dimethylethyl 6-chloro-4-hexenoate
n-Butyl lithium (1.6M; 6ml) was added over 5 min, under nitrogen, to a stirred, cooled (-10°) solution of cyclohexyl isopropyl amine (1.64ml) in dry tetrahydrofuran (10ml). After 5min the solution was cooled to -78° and after a further 15 min., t-butyl acetate (1.35ml) was added over 5min. After 20min., cis-1,4-dichloro-2-butene (4ml) was added and the mixture allowed to warm to 10° over 4h. The mixture was diluted with hydrochloric acid (1N; 25ml) and extracted with ether (2x30ml). The extracts were washed with hydrochloric acid (1N; 20ml), brine (20ml) and sodium bicarbonate (8%; 20ml), dried and evaporated in vacuo to leave a pale yellow oil. The excess dichlorobutene was removed by Kugelrohr distillation (90°/13mm) to leave a yellow oil. Flash column chromatography on silica eluting with light petroleum (40-60°), increasing to dichloromethane in petrol and finally dichloromethane, gave the title compound (0.66g) as a colourless liquid. T.l.c. SiO$_2$ [dichloromethane] Rf 0.48.

Example 1

[1R-[1α(Z), 2β,5β]]-6-[[2-(Hexahydro-1H-azepin-1-yl)-5-[[2'-(hydroxymethyl)[1,1'-biphenyl]-4-yl]methoxy]cyclopentyl]oxy]-4-hexenoic acid
To a solution of Intermediate 9 (250mg) in dimethoxyethane (8.5ml) was added sodium carbonate solution (2N:3.5ml), 1,3-dihydro-1-hydroxy-2,1-benzoxaborole (170mg) and tetrakistriphenylphosphine palladium (0) (23mg). The mixture was heated at reflux under nitrogen for 3h. After cooling, the solution was diluted with sulphuric acid (1N;70ml), washed with ether, neutralised (4N; sodium hydroxide solution followed by pH 6.5 buffer) and extracted into dichloromethane (3 x 50ml). Combined extracts were dried (MgSO$_4$) and evaporated to give a solid. Purification by chromatography [Fluka HF$_{254}$ SiO$_2$] eluting with dichlorometh-ane:ethanol:ammonia (100:100:1) gave the title compound (190mg) as a white foam. T.l.c. SiO$_2$ [dichloromethane: ethanol (1:1), 1% NH$_3$] Rf 0.55. [α]$_D^{20}$ = +27.88° (chloroform). I.r. (Nujol) 3300, 1720, 1575cm$^{-1}$.

Example 2

[1S-[1α(Z),2β,5β]]-6-[[2-(Hexahydro-1H-azepin-1-yl)-5-[[2'-(hydroxymethyl)[1,1'-biphenyl]-4-yl]methoxy]cyclopentyl]oxy]-4-hexenoic acid
Using Intermediate 10 (250mg) and 1,3-dihydro-1-hydroxy-2,1-benzoxaborole (170mg) and identical conditions to those employed in Example 1 above except that additional chromatography was required using triethylamine in place of ammonia gave the title compound (150mg) as a white foam. T.l.c. SiO$_2$ [dichloromethane:ethanol (1:1), 1% NH$_3$] Rf 0.55. [α]$_D^{20}$ = -29.77° (chloroform). I.r. (Nujol) 3300, 1720, 1575cm$^{-1}$.

Pharmaceutical examples of parenteral injections/infusions comprising the hydrochloride salt of Compound A

| (i) Hydrochloride salt of Compound A equivalent to 50 mg base | | | |
|---|---|---|---|
| β-Cyclodextrin hydrate | 143mg | 166mg | 238mg |
| Sodium hydroxide solution | to pH7 | to pH7 | to pH7 |
| Water suitable for injection | to 50ml | to 50ml | to 50ml |

The hydrochloride salt of Compound A was dissolved in 35ml water suitable for injection and the β-cyclodextrin was added. This solution was titrated to pH7 with 0.02M sodium hydroxide solution and then adjusted to volume with water suitable for injection.

The solution may then be sterilised by filtration and filled into vials or ampoules.

| (ii) Hydrochloride salt of Compound A equivalent to 50mg base | |
|---|---|
| β-Cyclodextrin hydrate | 166mg |
| Sodium chloride | 450mg |
| pH7.0 phosphate buffer | 2.5ml |
| Sodium hydroxide solution | to pH7 |
| Water suitable for injection | to 50ml |

The hydrochloride salt of Compound A was dissolved in approximately 25ml water suitable for injection. The β-cyclodextrin was dissolved therein and the resulting solution was titrated to pH6 with 0.02M sodium hydroxide solution and the phosphate buffer added. The sodium chloride was added to the solution and the pH adjusted to pH7 with sodium hydroxide. The solution was made up to volume with water suitable for injection. A sample of this solution was filled into a glass vial which was sealed with a rubber plug and metal overseal. This was then autoclaved.

| (iii) Hydrochloride salt of Compound A equivalent to 50mg base | |
|---|---|
| Hydroxypropyl-β-cyclodextrin | 170mg |
| Mannitol | 2.5g |
| pH 6.0 phosphate buffer | 5.0ml |
| Sodium hydroxide solution | to pH 6 |
| Water suitable for injection | to 50ml |

The hydrochloride salt of Compound A was dissolved in approximately 25ml water suitable for injection and the hydroxypropyl-β-cyclodextrin was added. The mannitol was then added and the solution titrated to pH 6 with 0.02M sodium hydroxide solution. The phosphate buffer solution was added and the solution adjusted to volume with water suitable for injection. The solution was then filtered and filled into glass vials which were sealed with rubber plugs and metal overseals. These were then autoclaved.

(iv) Hydrochloride salt of Compound A equivalent to 50mg base

| | |
|---|---|
| β-Cyclodextrin hydrate | 166mg |
| Mannitol | 2.5g |
| Sodium acid phosphate | 46mg |
| Disodium phosphate, anhydrous | 5mg |
| Sodium hydroxide solution | to pH 6 |
| Water suitable for injection | to 50ml |

The hydrochloride salt of Compound A was dissolved in approximately 25ml water suitable for injection. The β-cyclodextrin and mannitol were dissolved therein and the solution titrated to pH 6 with 0.02M sodium hydroxide solution. The sodium acid phosphate and anhydrous disodium phosphate were dissolved in water suitable for injection. This solution was added to the bulk solution which was made up to volume with water suitable for injection. The solution was filtered and filled into glass ampoules which were sealed and then autoclaved.

(v)

| | Cyclodextrin | | | |
|---|---|---|---|---|
| | | | | Mixture |
| | α | γ | | β + γ |
| Hydrochloride salt of Compound A equivalent to 50mg base | | | | |
| Cyclodextrin | 143mg | 190mg | 119mg | 136mg |
| Mannitol | 2.5g | 2.5g | 2.5g | |
| pH 6.0 Phosphate buffer | 5.0ml | 5.0ml | 5.0ml | |
| Sodium hydroxide solution | to pH 6 | to pH 6 | to pH6 | |
| Water suitable for injection | to 50ml | to 50ml | to 50ml | |

The hydrochloride salt of Compound A was dissolved in approximately 25ml water suitable for injection and the cyclodextrin(s) was (were) added. The mannitol was then added and the solution titrated to pH 6 with 0.02M sodium hydroxide solution. The phosphate buffer solution was added and the solution was adjusted to volume with water suitable for injection. The solution was then filtered and filled into glass vials which were sealed with rubber plugs and metal overseals.

Pharmaceutical example of an oral syrup comprising the hydrochloride salt of Compound A

| | |
|---|---|
| Hydrochloride salt of Compound A equivalent to 2.5mg base | |
| β-cyclodextrin hydrate | 9mg |
| Citric acid | to pH 4.5 |
| Methyl hydroxybenzoate sodium | 5mg |
| Propyl hydroxybenzoate sodium | 2mg |
| Liquid orange flavour | qs |
| Sucrose | 3.25g |
| Purified water | to 5.0ml |

Dissolve the sucrose in a minimum quantity of water. Add the hydrochloride salt of Compound A and then the β-cyclodextrin with stirring; adjust the pH to 4.5 with citric acid. With continued stirring add a solution of the hydroxybenzoates and lastly the flavour. Adjust almost to volume with water and stir. Check the pH and adjust to 4.5 with citric acid if necessary. Make up to volume with water.

## Claims

1. Use of TP-receptor antagonists in the manufacture of medicaments for the treatment or prophylaxis of conditions caused or exacerbated by myofibroblast cell contraction.

2. A use as claimed in Claim 1 in the manufacture of a medicament for the treatment or prophylaxis of conditions caused or exacerbated by myofibroblast cell contraction at a site of tissue damage.

3. A use as claimed in Claim 1 or Claim 2 in which the TP-receptor antagonist is [1R-[1α(Z),2β,3β,5α]]-(+)-7-[5-[[(1,1'-biphenyl)-4-yl] methoxy]-3-hydroxy-2-(1-piperidinyl)cyclopentyl]-4-heptenoic acid (Compound A) or a physiologically acceptable salt, solvate or cyclodextrin complex thereof.

4. A use as claimed in Claim 3 in which Compound A is in the form of its hydrochloride salt.

5. A use as claimed in Claim 1 or Claim 2 in which the TP-receptor antagonist is [1α(Z),2β,5β]--(±)-6-[[2-(hexahydro-1H-azepin-1-yl)-5-[[2'-(hydroxymethyl)[1,1'-biphenyl]-4-yl]methoxy]cyclopentyl]oxy]-4-hexenoic acid (Compound B) or the 1R or 1S-isomer thereof.

6. A use as claimed in Claim 5 in which the TP-receptor antagonist is the 1R-isomer of Compound B.

7. A use as claimed in Claim 5 in which the TP-receptor antagonist is the 1S-isomer of Compound B.

8. A use as claimed in any preceding claim in which the medicament claimed is in a form suitable for oral or parenteral administration.

9. A skin graft including a TP-receptor antagonist (as claimed in any of Claims 1 to 7) in an amount sufficient to inhibit or reduce the contractibility of myofibroblast cells at the site of the skin graft operation.